# EUROPEAN PATENT SPECIFICATION

(11) **EP 2 265 202 B1**
(45) Date of publication and mention of the grant of the patent: **29.08.2012**
(21) Application number: 09735637.2
(22) Date of filing: 22.04.2009
(51) Int. Cl.: A61B 17/70

(54) **BONE FIXATION ELEMENT WITH REDUCTION TABS**
KNOCHENFIXIERUNGSELEMENT MIT REDUZIERUNGSLASCHEN
ÉLÉMENT DE FIXATION OSSEUSE POURVU DE PATTES DE RÉDUCTION

(30) Priority: 22.04.2008 US 47025
(43) Date of publication of application: 29.12.2010
(73) Proprietor: Synthes GmbH, 4436 Oberdorf (CH)
(72) Inventor: STIHL, Pascal, CH-2540 Grenchen (CH); RUTSCHMANN, Helmut, 79576 Weil am Rhein (DE)
(74) Representative: Lusuardi, Werther
(86) International application number: PCT/US2009/041414
(87) International publication number: WO 2009/132110

(56) References cited:
- DE-C1- 4 436 262
- US-A1- 2005 273 101
- US-A1- 2006 025 771
- US-A1- 2006 036 252
- US-A1- 2006 058 794

## Description

The invention relates to a bone fixation element, according to the preamble of claim 1.

It is often necessary due to various spinal disorders to surgically correct and stabilize spinal curvatures, or to facilitate spinal fusion through an open approach or through a minimally invasive approach. Numerous systems for treating spinal disorders have been disclosed. One known method involves a pair of elongated members, typically rods, longitudinally fixed to the posterior spine on either side of spinous processes of the vertebral column. Each rod is attached to various vertebrae along the length of the spine by engaging bone fixation elements to the vertebra and fixing a rod to the bone fixation elements. The bone fixation elements commonly include a U-shaped rod-receiving channel for receiving the rod therein. Moreover, the rod-receiving channel often interacts with a locking cap to clamp and fix the position of the rod with respect to the bone fixation element.

Surgeons may have difficulty aligning the rod(s) within the rod-receiving channels formed in the body of the bone fixation elements. For example, the body of the bone fixation elements may be out of vertical and/or horizontal alignment with one another due to the curvature of the spine or the size and shape of each vertebra.

Thus, it is desirable to construct an apparatus to facilitate insertion of the longitudinal rods into the rod-receiving channels formed in the bone fixation elements.

From US 2006/036252 [Baynham et al.] a polyaxial screw is known based on the preamble of claim 1.

From US 2005/273101 [Schumacher] a bone screw and osteosynthesis device is known. From US 2006/025771 [Jackson] a helical reverse angle guide and advancement structure with break-off extensions is known. From US 2006/058794 [Jackson] a helical guide and advancement flange with break-off extension is known. DE 4436262 [Schäfer] discloses a reposition screw, in particular for vertebrae and from US 2007/0167948 [Abdou] devices and methods for inter-vertebral orthopedic devices placement are known.

A further anchoring element comprising a bone screw and a receiving member for connecting the bone screw to a rod is known from US 2002/143341 [Biedermann et al.].

A preferred embodiment of the present invention is directed to a bone fixation element for use in a posterior spinal fixation procedure to interconnect a longitudinal rod with a patient's vertebral body. The bone fixation element includes a body having a pair of spaced apart arms defining a rod-receiving channel and a pair of reduction tabs operatively coupled to the spaced apart arms to facilitate insertion of the longitudinal rod into the rod-receiving channels. In order to facilitate vertical reduction of the longitudinal rod into the rod-receiving channel, the bone fixation element includes a pair of reduction tabs extending therefrom to temporarily extend the overall height of the bone fixation element. The reduction tabs define a rod-receiving channel that aligns with and/or temporarily extends the rod-receiving channel formed in the bone fixation element.

The reduction tabs are preferably integrally formed with the bone fixation element. The reduction tabs preferably include a break-off point and/or region so that after the longitudinal rod has been inserted into the rod-receiving channel of the bone fixation element, the reduction tabs can be removed leaving in place the bone fixation element and longitudinal rod. The break-off points or regions are configured as an area of weakness formed in the reduction tabs so that the reduction tabs will break or rupture when subjected to high stresses, such as by stresses induced by the surgeon in order to break or sever the reduction tabs from the bone fixation element so that the reduction tabs can be removed from the patient's body after the longitudinal rod has been seated and secured within the rod-receiving channel.

The break-off points or regions may be configured as a groove. Preferably, each of the reduction tabs includes a pair of break-off grooves, one formed along the outer surface of the reduction tabs and one formed along the inner surface of the reduction tabs. The outer break-off groove is preferably located distally of the inner break-off groove. By incorporating inner and outer break-off grooves, the fracture line and surface may be controlled to substantially reduce potentially sharp edges as a result of the reduction tabs being broken or ruptured from the body.

Alternatively, the break-off points or regions may be configured as a pair of connection points on either side of a through slot, wherein the through slot extends from an outer surface of the tabs to an inner surface of the tabs.

The reduction tabs may also include a cap for coupling to a proximal end of the reduction tabs. The cap preferably includes at least one shoulder to enable a user to attach one or more instruments to the bone fixation element. The cap may also include a drive surface for receiving a corresponding tip formed on a drive tool for applying a counter torque force.

The bone fixation element including reduction tabs may be provided as a part of a system including a reduction tab removal instrument for severing the reduction tabs from the bone fixation element after the longitudinal rod has been seated within the rod-receiving channel of the bone fixation element. The reduction tab removal instrument may include an outer shaft including a plurality of expandable prongs on a distal end thereof, an inner shaft slidably disposed within the outer shaft and a handle operatively coupled to the outer and inner shafts for operatively moving the inner shaft with respect to the outer shaft such that movement of the inner shaft with respect to the outer shaft causes the plurality of expandable prongs to radially expand. The expandable prongs may include a wedge shaped portion for contacting the through slot formed in the break-off region.

The inner surface of the reduction tabs and the inner surface of the spaced apart arms preferably include a plurality of threads for engaging an externally threaded locking cap. More preferably, the threads have a negative thread profile to limit splaying.

### BRIEF DESCRIPTION OF THE SEVERAL VIEWS OF THE DRAWINGS

The foregoing summary, as well as the following detailed description of the preferred embodiments of the application, will be better understood when read in conjunction with the appended drawings. For the purposes of illustrating the preferred bone fixation elements and surgical method for inserting the bone fixation elements of the present application, there are shown in the drawings preferred embodiments. It should be understood, however, that the application is not limited to the precise arrangement and instrumentalities shown. In the drawings:

Fig. 1 is a cross-sectional view of a first preferred embodiment of a bone fixation element of the present invention, with a longitudinal rod positioned in a rod-receiving channel of the bone fixation element in an insertion position;

Fig. 2 is a cross-sectional view of the bone fixation element shown in Fig. 1, the longitudinal rod located in a seated position;

Fig. 3 is a cross-sectional view of the bone fixation element shown in Fig. 1, with reduction tabs of the bone fixation element being removed from a body in an implanted configuration and the longitudinal rod located in the seated position;

Fig. 4 is a cross-sectional view of the body of the bone fixation element of Fig. 1;

Fig. 5 is a detailed, cross-sectional view of a break-off region of the body of Fig. 4, taken from within circle 5 of Fig. 4;

Fig. 6 is a detailed, cross-sectional view of a thread profile on an inner surface of the body of Fig. 4, the thread profile taken from within circle 6 of Fig. 4.

Fig. 7 is a front elevational view of a second preferred embodiment of a bone fixation element of the present invention with a longitudinal rod positioned in the seated position and a first preferred cap mounted to a proximal end;

Fig. 8 is a front elevational view of a third preferred embodiment of a bone fixation element of the present invention with a longitudinal rod positioned in the seated position and a second preferred cap mounted to a proximal end;

Fig. 9 is a magnified, side perspective view of the bone fixation element of Fig. 7 with an exemplary break-off region;

Fig. 10A is a front elevational view of a reduction tab removal tool that may be used in connection with the bone fixation element of Fig. 7; and

Fig. 10B is a magnified front elevational view of a distal end of the reduction tab removal tool of Fig. 10A, taken from within circle 10B of Fig. 10A.

### DETAILED DESCRIPTION OF THE INVENTION

Certain terminology is used in the following description for convenience only and is not limiting. The words "right", "left", "lower" and "upper" designate directions in the drawings to which reference is made. The words "inwardly" and "outwardly" refer to directions toward and away from, respectively, the geometric center of the bone fixation element, instruments and designated parts thereof. The words, "anterior", "posterior", "superior", "inferior", "medial", "lateral" and related words and/or phrases designate preferred positions and orientations in the human body to which reference is made and are not meant to be limiting. The terminology includes the above-listed words, derivatives thereof and words of similar import.

Certain exemplary embodiments of the invention will now be described with reference to the drawings. In general, such embodiments relate to a bone fixation element 10, 10', 10" by way of non-limiting example, a bone fixation element 10, 10', 10" for use in a posterior spinal fixation procedure to interconnect a longitudinal rod 45 with a patient's vertebra V. The invention may have other applications and uses and should not be limited to the structure or use described and illustrated. The same reference numerals will be utilized throughout the application to describe similar or the same components of each of the preferred embodiments of the bone fixation elements and instruments described herein and the descriptions will focus on the specific features of the individual embodiments that distinguish the particular embodiment from the others.

As will be described in greater detail below, the bone fixation element 10, 10', 10" preferably includes a bone anchor 12 for securing the bone fixation element 10, 10', 10" to a patient's vertebra V, a body 20 having a rod-receiving channel 26 for receiving a longitudinal rod 45, and a locking cap 40 for securing the rod 45 in the rod-receiving channel 26 in an implanted configuration. The bone fixation element 10, 10', 10" also includes a pair of reduction tabs 100, 102 extending from a break-off point or region 150 to a proximal end 106 of the body 20. The reduction tabs 100, 102 facilitate or guide insertion of the longitudinal rod 45 into the rod-receiving channels 26. The break-off point and/or region 150 facilitates removal of the reduction tabs 100, 102 from the arms 28, 30 of the body 20 after the longitudinal rod 45 has been inserted in the rod-receiving channel 26 of the bone fixation element 10, 10', 10" in a seated position.

While the bone fixation element 10, 10', 10" will be described as and may generally be used in the spine (for example, in the lumbar, thoracic or cervical regions), those skilled in the art will appreciate that the bone fixation element 10, 10', 10" may be used for fixation of other parts of the body such as, for example, joints, long bones or bones in the hand, face, feet, extremities, cranium, *etc*.

As generally understood by one of ordinary skill in the art, the longitudinal rod 45 may include, but not limited to, a solid rod, a non-solid rod, a flexible or dynamic rod, *etc*. It will be understood by one having ordinary skill in the art that the bone fixation element 10, 10', 10" is not limited to mounting to any particular type of longitudinal rod 45.

As generally understood by one of ordinary skill in the art, the bone fixation element 10, 10', 10" is used generally and may include, but are not limited to, poly-axial or mono-axial pedicle screws, hooks (both mono-axial and poly-axial) including pedicle hooks, transverse process hooks, sublaminar hook, or other fasteners, clamps or implants.

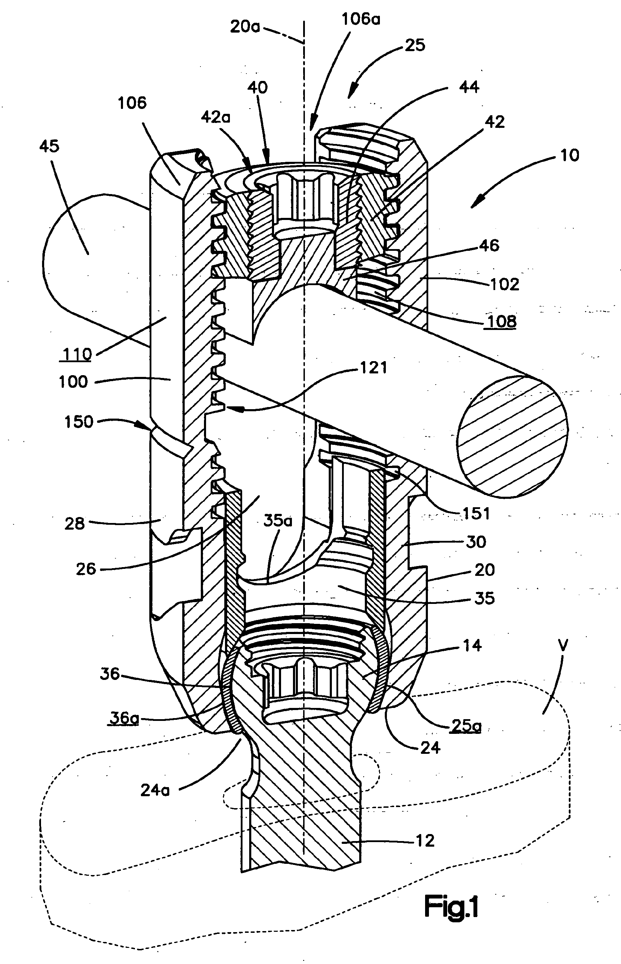

Referring to Figs. 1-3, in the first preferred embodiment, the bone fixation element 10 includes the bone anchor 12 (shown as a bone screw) having an enlarged, spherically-shaped head portion 14, the locking cap 40, a sleeve 35, a bushing 36 and the body 20 (shown as a top loading body) having the proximal end 106, a distal end 24, the rod-receiving channel 26 (shown as a top loading U-shaped rod-receiving channel) defining the pair of spaced apart arms 28, 30 and the reduction tabs 100, 102 extending from the arms 28, 30. The locking cap 40 is preferably a two-step locking cap 40, as will be described in greater detail below. The sleeve 35 and bushing 36 are preferably slidably disposed within the body 20 and at least a portion of the bushing 36 is preferably slidably disposed within at least a portion of the sleeve 35. In use, the enlarged head portion 14 of the bone anchor 12 may be received within an inner spherical cavity formed in the bushing 36, which may be received within the distal end 24 of the body 20 so that the bone anchor 12 can poly-axial rotate with respect to the bushing 36, which can poly-axial rotate with respect to the body 20. Alternatively, the bone anchor 12 may be formed integral with the body 20 to form a monolithic structure which is referred to as a mono-axial pedicle screw or hook.

The body 20 preferably includes a plurality of threads 121 on an inner surface of the reduction tabs 100, 102 and the arms 28, 30 for threadably receiving the locking cap 40. Although, as will be generally appreciated by one of ordinary skill in the art, other engagements are envisioned including, but not limited to, an externally threaded body for threadably engaging an internally threaded locking cap, a cam lock, *etc.* The proximal and distal ends 106, 24 of the body 20 preferably include a top opening 106a and a bottom opening 24a, respectively. A longitudinal bore 25 extends from the top opening 106a to the bottom opening 24a. The enlarged head portion 14 of the bone anchor 12, the bushing 36, the sleeve 35, the locking cap 40 and at least a portion of the longitudinal rod 45 are receivable in the longitudinal bore 25. The longitudinal bore 25 preferably includes a curvate, preferably frusta-spherical, contacting surface 25a adjacent to the bottom opening 24a for contacting and receiving a curvate, preferably frusta-spherical, outer surface 36a of the bushing 36 so that the bushing 36, and hence the bone anchor 12 are polyaxially rotatable with respect to the body 20.

The bushing 36 and the body 20 are preferably configured so that the lower end of the bushing 36 sits below the distal end 24 of the body 20. As a result of the lower end of the bushing 36 sitting lower than the distal end 24 of the body 20, the bushing 36 enables the bone anchor 12 to rotate polyaxially with respect to the body 20 to a greater degree than polyaxially pedicle screws having a bushing completely positioned within their body. In addition, since the enlarged head portion 14 of the bone anchor 12 is able to rotate with respect to the bushing 36 and the bushing 36 is able to rotate with respect to the body 20, the combination of bushing 36 and bone anchor 12 allows the bone anchor 12 to rotate to a relatively large degree.

The locking cap 40 is in the form of a two step locking cap 40 including an externally threaded outer part 42 with an internal bore 42a for threadably receiving an externally threaded inner part 44. A saddle 46 is preferably coupled to the inner part 44 of the locking cap 40. The saddle 46 is preferably rotatably coupled to the inner part 44 of the locking cap 40 so that the saddle 46 can contact the top surface of the longitudinal rod 45 while the locking cap 40 is being rotated.

Exemplary embodiments of pedicle screws include those described in International Patent Application No. PCT/US2008/070670, filed on July 21, 2008, and published as WO 2009/015100 entitled "polyaxial Bone Fixation Element", International Patent Application No. PCT/US2006/015692, filed on April 25, 2006, and published as WO 2006/116437 entitled "Bone Anchor with Locking Cap and Method of Spinal Fixation", and International Patent Application No. PCT/CH1997/00236, filed on June 16, 1997, as published as WO 1998/052482 entitled "Device for Connecting a Longitudinal Support with a Pedicle Screw", the contents of which are hereby incorporated by reference in their entirety. It should be understood however that the present invention is not limited in use to any particular type of pedicle screw.

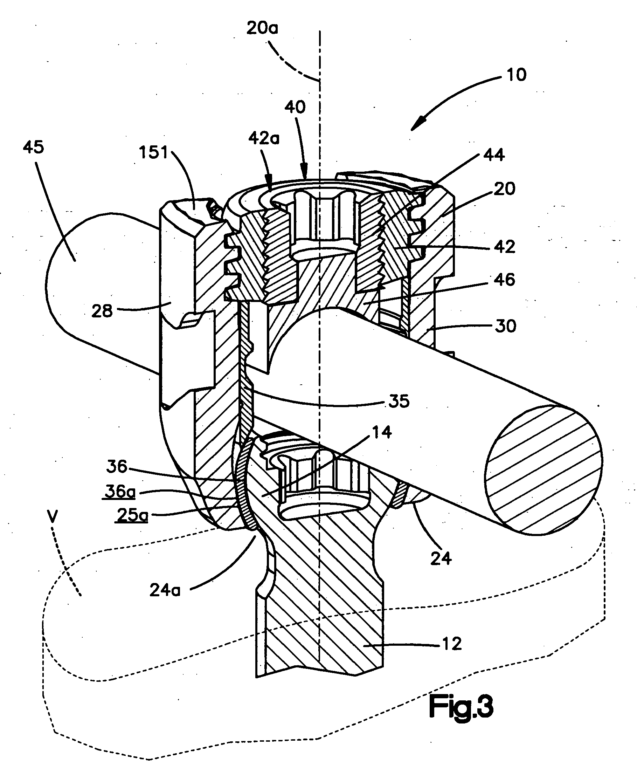

Referring to Figs. 1-4, the bone fixation element 10 of the first preferred embodiment may have a low profile in an implanted configuration (Fig. 3) so that the height of the bone fixation element 10 is minimized. The overall height of the body 20 in the implanted configuration may be minimized so that, once engaged, the bone fixation element 10 extends a limited distance away from the vertebra V. The body 20 of the bone fixation element 10 preferably includes a pair of reduction tabs 100, 102 extending from the pair of spaced apart arms 28, 30 to temporary extend the overall height of the bone fixation element 10 and facilitate insertion of the rod 45 into the rod-receiving channel 26 of the bone fixation element 10 during an insertion procedure. For example, the reduction tabs 100, 102 may have a length of about twenty millimeters (20 mm), although it is envisioned that the reduction tabs 100, 102 may be longer or shorter. Thereafter, once the longitudinal rod 45 is received within the rod-receiving channel 26 of the body 20 and the locking cap 40 is in the seated position, the reduction tabs 100, 102 may be broken off, removed and/or disposed, leaving behind the lower portion of the body 20, the locking cap 40 and the clamped rod 45 in the implanted configuration (Fig. 3).

The reduction tabs 100, 102 are preferably integrally formed with the pair of spaced apart arms 28, 30 to form the body 20. Although, as will be generally appreciated by one of ordinary skill in the art, the reduction tabs 100, 102 may be coupled to the pair of arms 28, 30 by any means known in the art including, but not limited to, adhesive bonding, mechanical fastening, clamping, *etc.* As shown, the reduction tabs 100, 102 generally define the rod-receiving channel 26 formed in the body 20 between the break-off region 150 and the proximal end 106. The reduction tabs 100, 102 preferably includes an inner surface 108 and an outer surface 110. The inner surface 108 of the reduction tabs 100, 102 preferably includes threads 121. More preferably, the threads 121 begin at or near the proximal end 106 and extend substantially the entire length of the reduction tabs 100, 102. The threads 121 preferably have a thread profile that matches the thread profile of the threads 21 formed on the inner surface of the lower portion of the body 20 and the thread profile on the outer surface of the locking cap 40. More preferably, as will be described in greater detail below, the threads on the inner surface of the body 20 and the outer surface of the locking cap 40 have a negative thread profile.

The break-off point(s) or region(s) 150 generally separates the lower portion of the body 20 and the reduction tabs 100, 102. More preferably the reduction tabs 100, 102 and the pair of spaced apart arms 28, 30 are preferably integrally formed as a portion of the body 20. The break-off points or regions 150 are preferably an area of weakness formed at a distal end of the reduction tabs 100, 102 so that the reduction tabs 100, 102 are breakable from the spaced apart arms 28, 30 when subjected to high stresses, such as by stresses induced by the surgeon in order to break or dislodge the reduction tabs 100, 102 from the spaced apart arms 28, 30. The reduction tabs 100, 102 are preferably designed to break off such that they can be, partially or completely, removed from the patient's body after the longitudinal rod 45 has been seated and secured within the rod-receiving channel 26 in the implanted configuration. The reduction tabs 100, 102 may be severed from the spaced-apart arms 28, 30 via pliers, a break-off tool, by grasping and applying forces to the proximal end 106, by hand or in numerous other ways that permit application of rupturing forces at the break-off points or regions 150.

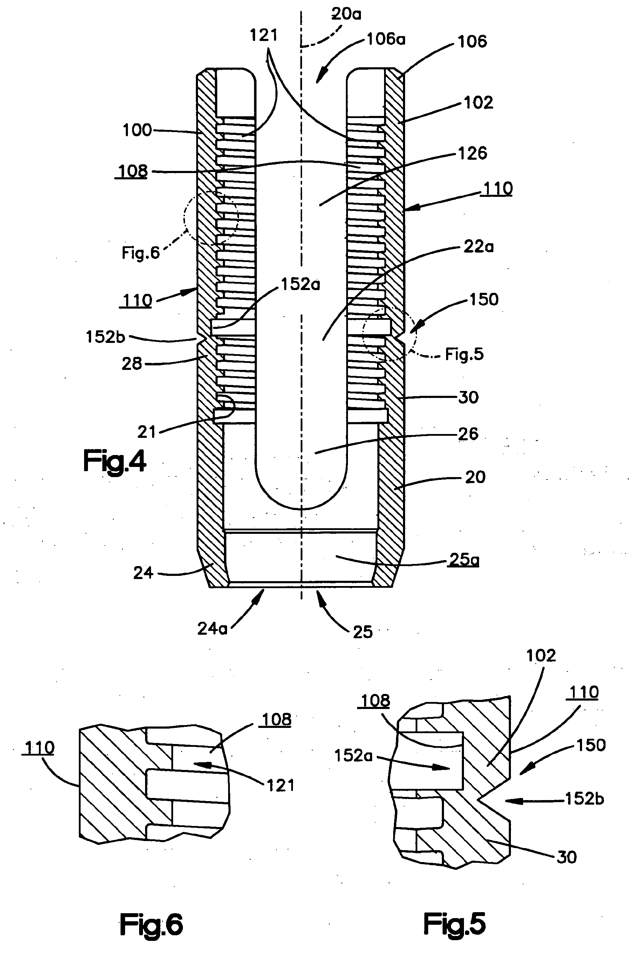

Referring to Figs. 4 and 5, the break-off points or regions 150 of the first preferred embodiment are configured as a pair of grooves 152a, 152b. Alternatively, the break-off points or regions 150 may be configured as a single weakened region, thinned-out area, *etc.* More preferably, the body 20 includes the pair of break-off grooves 152a, 152b including an outer break-off groove 152b formed along the outer surface 110 of the reduction tabs 100, 102 and an inner break-off groove 152a formed along the inner surface 108 of the reduction tabs 100, 102. The outer break-off groove 152b is preferably located distally of the inner break-off groove 152a, but is not so limited and the outer break-off groove 152b may be located proximally in comparison to the inner break-off groove 152a or the inner and outer break-off grooves 152a, 152b may be formed generally at the same level along the length of the body 20. By incorporating inner and outer break-off grooves 152a, 152b, a fracture line 151 location and configuration can be influenced. Specifically, the shape and location of the inner and outer break-off grooves 152a, 152b of the first preferred embodiment are arranged to limit any potentially sharp edges as a result of the reduction tabs 100, 102 being broken or ruptured from the body 20.

The bone fixation element 10 of the first preferred embodiment is assembled by inserting the enlarged head portion 14 of the bone anchor 12 into the inner, frusta-spherically-shaped cavity of the bushing 36. The bushing/screw assembly is then inserted into the body 20 through the top opening 106a until the frusta-spherical outer surface of the bushing 36 contacts the frusta-spherical inner surface 25a of the body 20 adjacent to the distal end 24. The sleeve 35 is then inserted into the body 20 through the top opening 106a so that the sleeve 35 is positioned on top of the bushing 36. The top surface of the sleeve 35 preferably includes a seat 35a for receiving the longitudinal rod 45 in the seated position. The body/sleeve/bushing/bone anchor - assembly may be provided pre-assembled to surgeons so that, in use, a surgeon can implant the assembly into the patient's vertebra V.

In use, the bone anchor 12 may be secured to a patient's bone, preferably the patient's vertebra V, by driving the bone anchor 12 with a screwdriver or other similar screw driving device. With the bone anchor 12 engaged to the patient's bone, the surgeon is able to polyaxially rotate the body 20 with respect to the bone anchor 12 and with respect to the engaged bone so that the longiditudinal rod 45 can be inserted into the rod-receiving channels 126 defined by the reduction tabs 100, 102 of adjacent bone fixation elements 10. The longitudinal rod 45 may or may not be seated within the rod-receiving channel 26 of the bone fixation element 20.

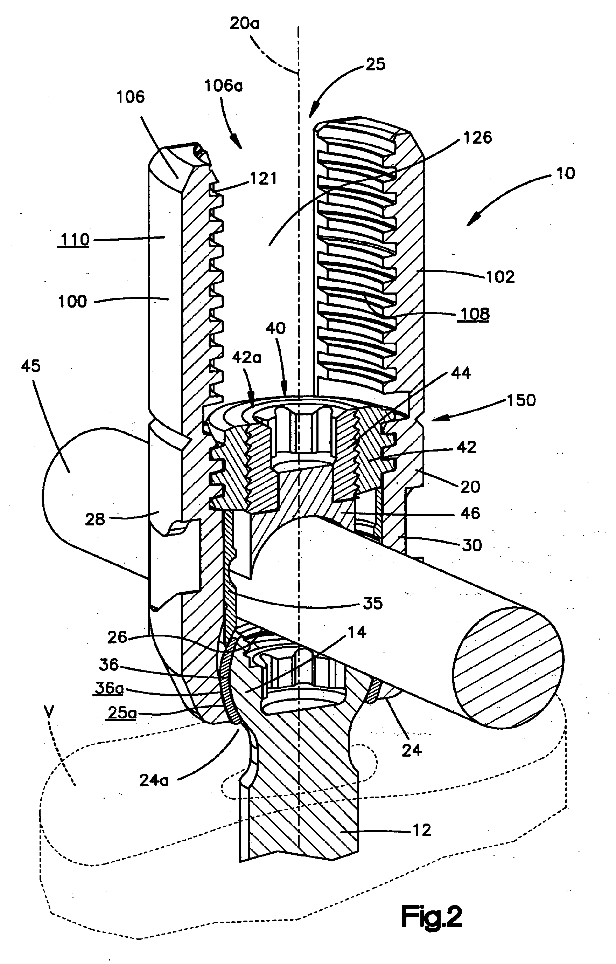

Once the longitudinal rod 45 is received within the rod-receiving channel 126, the locking cap 40 is threadably coupled along the inner surface 108 of the reduction tabs 100, 102. Thereafter, rotation of the locking cap 40 causes the bottom surface of the locking cap 40 to contact the top surface of the longitudinal rod 45, which is located within the rod receiving channel 126, thereby urging the longitudinal rod 45 towards the bottom opening 24a of the body 20 and into the rod-receiving channel 26 formed in the body 20, as generally depicted in Figs. 1 and 2

Continued rotation of the locking cap 40 toward the distal end 24 urges the locking cap 40 into the sleeve 35, thereby urging the sleeve 35 downwards into further contact with the bushing 36. Downward movement of the sleeve 35 causes the bushing 36 to radially compress against the enlarged head portion 14 of the bone anchor 12, which in turn, fixes the position of the bone anchor 12 with respect to the body 20.

The longitudinal rod 45 may still be movable with respect to the body 20 so that distraction and/or compression of the patient's adjacent vertebrae V may be accomplished, typically by applying distraction and/or compression to the bone anchors 12. Once the desired position of the adjacent vertebrae V is accomplished, the inner part 44 of the locking cap 40 is rotated with respect to the outer part 42 of the locking cap 40, urging the inner part 44 toward the distal end 24. Rotation of the inner part 44 of the locking cap 40 causes the inner part 44 of the locking cap 40 to urge the saddle 46 into contact with the longitudinal rod 45 and against the sleeve 35, which in turn fixes the position of the longitudinal rod 45 with respect to the body 20.

As generally shown in Fig. 3, once the longitudinal rod 45 is secured within the rod-receiving channel 26 of the bone fixation element 10, the reduction tabs 100, 102 are preferably removed by breaking the reduction tabs 100, 102 at the break-off points or regions 150, thereby leaving the lower portion of the body 20, the locking cap 40 and the clamped longitudinal rod 45 within the patient's body.

The bone fixation element 10 may be manufactured from any biocompatible material such as, but not limited to, titanium, titanium alloys, stainless steel, cobalt chromium, Nitinol, *etc*. Moreover, as will be generally understood by one of ordinary skill in the art, the bone fixation element 10 may be provided in any number of sizes and configurations depending on the size and configuration of the bone anchor 12, longitudinal rod 45 and/or on the type and location of the surgery being performed.

Referring to Fig. 6, the threads 21, 121 formed on the inner surface of the body 20 and the threads formed on the outer surface of the locking cap 40 preferably incorporate a negative thread profile. That is, the load flank and stab flank of the threads 21, 121 and the outer surface of the locking cap 40 are angled downwards with respect to a plane perpendicular to the longitudinal axis 20a of the body 20 and the locking cap 40. In the first preferred embodiment, the threads 21, 121 form an angle of negative ten degrees (-10°) with respect to a plane perpendicular to the longitudinal axis 20a of the body 20. The threads 21, 121 are not limited to the negative ten degree (-10°) angles and may form nearly any angle that permits engagement and driving of the locking cap 40 into the body 20. The negative thread profile is preferred to limit splaying of the arms 28, 30 and reduction tabs 100, 102 as the locking cap 40 is being urged downwardly toward the distal end 24.

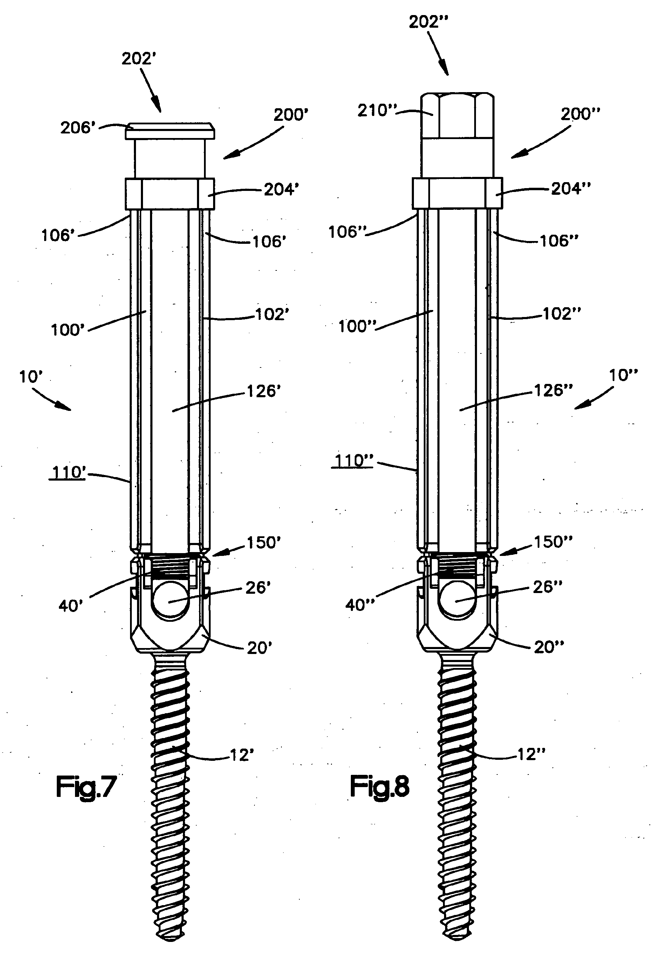

Referring to Fig. 7, a second preferred embodiment of the bone fixation element 10' is substantially similar to bone fixation element 10 of the first preferred embodiment and like elements are identified using like reference numerals with a prime symbol (') to indicate the second preferred embodiment. The bone fixation element 10' of the second preferred embodiment includes a proximal end cap 200'. The proximal end cap 200' connects the reduction tabs 100', 102' adjacent to the proximal end 106' of the body 20'. The cap 200' preferably includes an internal bore 202' that enables the locking cap 40' to pass therethrough. The cap 200' is preferably integrally formed with the reduction tabs 100', 102'. Alternatively, the cap 200' may be operatively connected to the reduction tabs 1.00', 102' by nearly any connection mechanism including, but not limited to, adhesive, bonding, mechanical fasteners, *etc*.

By providing the cap 200', splaying of the reduction tabs 100', 102', as a consequence of the locking cap 40' being rotated toward the distal end 24 to urge the longitudinal rod 45 into the rod-receiving channel 26' is preferably limited, thereby enabling the length of the reduction tabs 100', 102' to be increased. Overall lengthening of the reduction tabs 100', 102' may be beneficial for lumbar applications and in enabling the reduction tabs 100', 102' to be used in minimally invasive procedures for guiding a longitudinal rod 45 into the rod-receiving channel 26' of the bone fixation element 10', as will be described in greater detail below. For example, the reduction tabs 100', 102' may have a length of about one hundred millimeters (100 mm), although it is envisioned that the reduction tabs 100', 102' may be longer or shorter to accommodate various patient anatomy.

The cap 200' of the second preferred embodiment includes a distal shoulder 204' and a proximal shoulder 206'. The distal and proximal shoulders 204', 206' enables the user to attach one or more instruments to the bone fixation element 10' to improve control of the bone fixation element 10'. For example, the shoulders 204', 206' enable the user to attach a distraction/compression instrument or forceps to perform rod reduction, a counter-torque device, *etc.*

The preferred cap 200' also provides the bone fixation element 10' with a holding mechanism to facilitate percutaneous control of the rod-receiving channel 26', as will be described in greater detail below.

Referring to Fig. 8, a third preferred embodiment of the bone fixation element 10" is substantially similar to bone fixation element 10' of the second preferred embodiment and like elements are identified using like reference numerals with a double-prime symbol (") to indicate the third preferred embodiment. In the third preferred embodiment, the cap 200" includes a drive surface 210" for receiving a corresponding tip (not shown) formed on a drive tool (not shown). For example, the drive surface 210" may be in the form of an external hex for engaging a corresponding internal hex so that the user may apply a counter torque so that during final tightening of the locking cap 40", a counter-acting torque may be applied. The drive surface 210" may have any form now or hereafter known including, but not limited to, an internal hexagon, a star drive pattern, a Phillips head pattern, a slot for a screw driver, a threading for a correspondingly threaded post, *etc.*

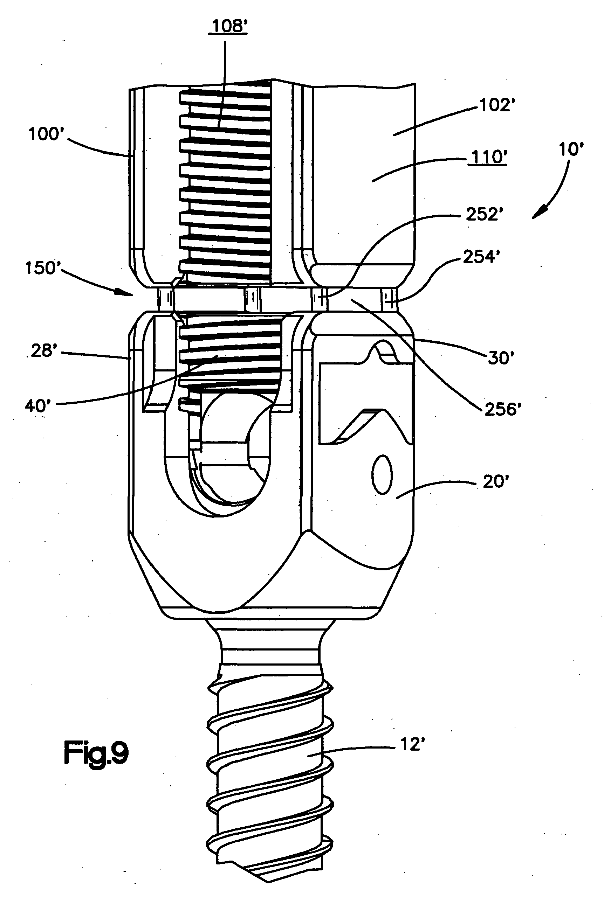

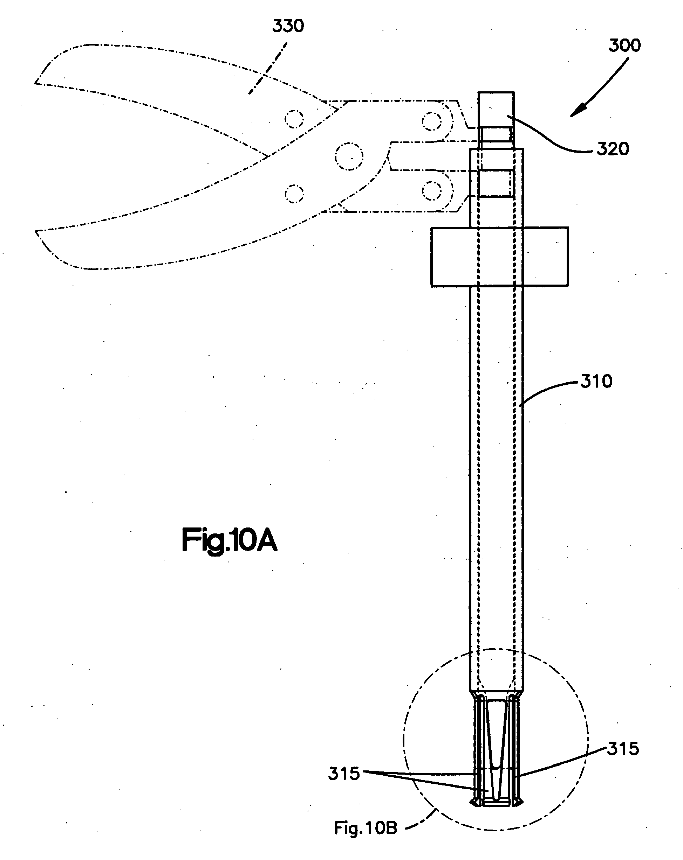

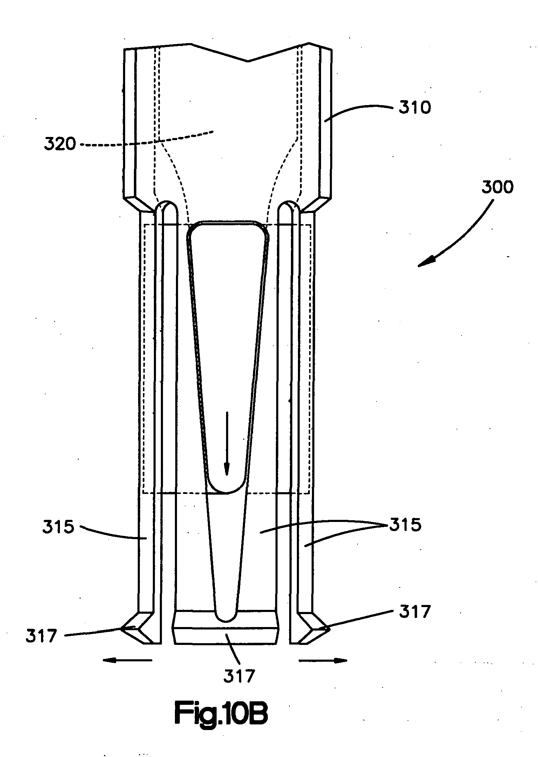

Referring to Figs. 9-10B, the break-off points or regions 150' of bone fixation element 10' of the second preferred embodiment may be configured as a pair of connection points 252', 254' on either side of a through slot 256'. In the second preferred embodiment, the proximal end cap 200' may increase the strength and stability of the break-off region 150' and increase the force required to remove the reduction tabs 100', 102'. Thus, to facilitate removal of the tabs 100', 102' from the bone fixation element 10' after the rod 45 is seated within the rod-receiving channel 26' of the bone fixation element 10', the break-off point or region 150' includes a through slot 256' for mating with a reduction tab removal instrument 300. The reduction tab removal instrument 300 preferably includes an outer shaft 310 including a plurality of expandable prongs 315 on a distal end thereof, an inner shaft 320 slidably disposed within the outer shaft 310 and a handle 330 operatively coupled to the outer and inner shafts 310, 320 for operatively moving the inner shaft 320 with respect to the outer shaft 310. The expandable prongs 315 preferably include a wedge-shaped portion 317 for contacting and/or engaging the through slot 256 formed in the break-off points or regions 150.

In use, after the longitudinal rod 45 has been properly secured within the rod-receiving channel 26' of the bone fixation element 10' via the locking cap 40', the outer and inner shafts 310, 320 of the reduction tab removal instrument 300 are inserted into the rod-receiving channel 126'. Preferably, the wedge-shaped portion 317 formed on the outer surface of the outer shaft 310 is placed into operative association with the through slot 256' of the break-off points or regions 150'. Thereafter, squeezing of the handles 330 causes the inner shaft 320 to move distally with respect to the outer shaft 310, which in turn causes the expandable prongs 315 formed on the distal end of the outer shaft 310 to radially expand resulting in the wedge shaped portions 317 pushing into or onto the through slots 256' resulting in the breaking and removal of the reduction tabs 100', 102'.

It should be noted that other instruments may be used to facilitate breaking and removal of the reduction tabs 100', 102'. For example, an instrument may be rotated so that wedge-shaped portions may shear the reduction tabs 100', 102' from the bone fixation element 10'.

In use, the bone fixation elements 10' are preferably implanted into the patent's vertebra V via a minimally invasive surgical technique. For example, the bone fixation element 10' may be inserted via a cannula. Once implanted, the cannula may be removed, leaving behind the bone fixation elements 10' including reduction tabs 100', 102'. Thereafter, the user may align the rod-receiving channels 26' of the bone fixation elements 10' so that the longitudinal rod 45 may be guided into place. Next, a rod-reducing instrument (not shown) may be operatively coupled to the reduction tabs 100', 102', and in particular to the cap 200'. The rod-reducing instrument may be used to reduce or seat the rod 45 into the rod-receiving channel 26' of the bone fixation element 10'. The locking cap 40' is inserted and rotated into place until the rod 45 is secured within the rod-receiving channel 26'. The reduction tab removal instrument 300 may then be inserted between the reduction tabs 100', 102' and activated to sever the reduction tabs 100', 102' from the bone fixation element 10'. Lastly, the reduction tabs 100', 102' are removed and the incision is closed.

Alternatively, the bone fixation elements 10 may be inserted into the patient's adjacent vertebrae V by forming an incision in the patient, positioning K-wires into the adjacent vertebra V to define trajectory, sequentially engaging a screwdriver (not shown) with the heads 14 and screwing the bone anchors 12 into the adjacent vertebra V along the K-wires and aligning the rod receiving channels 26 of adjacent bone fixation elements 10. The rod receiving channels 26 preferably extend out of the incision and are utilized to guide the longitudinal rod 45 into the lower portion of the body 20. A reduction tool may be utilized to reduce the rod 45 into the lower portion of the body 20 or locking caps 40 may be engaged with the inner threads 121 and driven toward the distal end 24 to move the rod 45 from the insertion position to the seated position. The locking caps 40 are urged toward the distal end 24 at least until the bushing 36 engages and locks the position of the bone anchors 12 relative to the bodies 20. One of the inner parts 44 is driven toward the distal end 24 to lock the rod 45 relative to the body 20 and the adjacent bone fixation element 10 is not final tightened such that compression or distraction may be performed on the adjacent vertebra. When the distraction or retraction is completed, the second inner part 44 is driven toward the distal end 24 to fix the rod 45 relative to both adjacent fixation elements. The reduction tabs 100, 102 are broken away from the lower portion of the body 20 at the break-off regions 150 and are removed from the patient's body. The procedure may be performed in a minimally invasive manner using percutaneous or mini-open incisions to receive a single bone fixation element 10 through the percutaneous incision or at least a pair of bone fixation elements 10 through the mini-open incision.

As will be appreciated by those skilled in the art, any or all of the components described herein may be provided in sets or kits so that the surgeon may select various combinations of components to perform a fixation procedure and create a fixation system which is configured specifically for the particular needs/anatomy of a patient. It should be noted that one or more of each component may be provided in a kit or set. In some kits or sets, the same device may be provided in different shapes and/or sizes.

## Claims

1. A bone fixation element (10) for interconnecting a rod (45) to a patient's bone, the bone fixation element (10) comprising:
a body (20) having spaced apart arms (28,30) defining a rod-receiving channel (26) therebetween, each of the arms (28,30) including a reduction tab (100,102) extending therefrom, the reduction tabs (100,102) separated from the arms (28,30) by inner and outer break-off regions (150), the inner break-off region formed on the inner surface of the body (20) and the outer break-off region formed on the outer surface of the body (20), inner threaded surfaces (121) formed along the length of the inner surface of the body (20), the body (20) having a longitudinal bore extending from a top opening formed in the body (20) to a bottom opening formed in the body (20);
a bushing (36) inserted in the longitudinal bore, including a lower end, and
an externally threaded locking cap (40) threadably engageable with the inner threaded surfaces, the reduction tabs (100,102) being separable from the spaced apart arms (28,30) at the inner and outer break-off regions (150),
**characterized in that**
A) the locking cap (40) is a two-step locking cap (40) including an externally threaded outer part (42) with an internal bore (42a) for threadably receiving an externally threaded inner part (44), whereby upon rotation of the cap (40) the outer part (42) of the locking cap (40) locks the bushing (36) while the inner part (44) of the locking cap (40) locks the rod (45);
B) the body (20) further includes a sleeve (35), the sleeve (35) and bushing (36) being slidably disposed within the longitudinal bore, at least a portion of the bushing (36) slidably disposed within the sleeve (35); and
C) the bushing (36) has an inner spherical cavity for slidably receiving a spherical screw head.

2. The bone fixation element of claim 1, wherein the reduction tabs are integrally formed with the spaced apart arms.

3. The bone fixation element of claim 2, wherein the inner and outer break-off regions are grooves.

4. The bone fixation element of claim 1, wherein the inner and outer break-off regions (150) in the form of grooves are not on the same level and preferably the outer break-off region is located distally relative to the inner break-off region. ".

5. The bone fixation element of claim 1, wherein the inner threaded surfaces have a negative thread profile.

6. The bone fixation element of claim 1, further comprising:
a bone anchor polyaxially disposed within a longitudinal bore extending from a top opening formed in the body to a bottom opening formed in the body.

7. The bone fixation element of claim 1, further comprising a bone anchor (12) having an enlarged, spherically-shaped head portion (14) enabling the bone anchor (12) to poly-axially rotate with respect to the bushing (36).

8. The bone fixation element of claim 7, wherein the bushing includes an inner frusta-spherical cavity for polyaxially receiving an enlarged head portion of the bone anchor.

9. The bone fixation element of claim 7, wherein the longitudinal bore includes a frusta-spherical contacting surface adjacent to the bottom opening for receiving an outer frusta-spherical surface formed on the bushing so that the bushing is polyaxially rotatable with respect to the body.

10. The bone fixation element of claim 9, wherein the bushing includes a lower end, the lower end of the bushing extending through the bottom opening of the body when the locking cap is threadably engaged with the inner threaded surfaces of the body.

11. The bone fixation element of claim 1, further comprising:
a cap mounted to a proximal end of the reduction tabs.

12. The bone fixation element of claim 11, wherein the cap includes a shoulder.

13. The bone fixation element of claim 11, wherein the cap includes a drive surface for engaging a drive tool for applying a counter torque force.

14. The bone fixation element of claim 11, wherein the inner and outer break-off regions are comprised of a pair of connection points on either side of a through slot, the through slot extending from the outer surface to the inner surface.

## Patentansprüche

1. Knochenfixationselement (10) zum Verbinden eines Stabs (45) mit einem Knochen eines Patienten, wobei das Knochenfixationselement (10) umfasst:
einen Körper (20) mit voneinander beabstandeten Armen (28, 30), welche dazwischen einen Kanal (26) zur Aufnahme eines Stabs definieren, wobei jeder der Arme (28, 30) einen sich von ihm ausdehnenden Reduktionslappen (100, 102) umfasst, wobei die Reduktionslappen (100, 102) durch innere und äussere Sollbruchbereiche (150) von den Armen (28, 30) separiert sind, wobei der innere Sollbruchbereich an der Innenfläche des Körpers (20) ausgebildet ist und der äussere Sollbruchbereich an der Aussenfläche des Körpers (20) ausgebildet ist, wobei entlang der Länge der Innenfläche des Körpers (20) innere gewindete Oberflächen (121) ausgebildet sind, und wobei der Körper (20) eine Längsbohrung aufweist, welche sich von einer im Körper (20) gebildeten oberen Öffnung zu einer im Körper (20) gebildeten unteren Öffnung erstreckt;
eine Büchse (36), welche in die Längsbohrung eingeführt ist und ein unteres Ende umfasst; und
eine Verriegelungskappe (40) mit einem Aussengewinde, welche mit den inneren gewindeten Oberflächen in einen Gewindeeingriff bringbar ist, wobei die Reduktionslappen (100, 102) an den inneren und äusseren Sollbruchbereichen von den voneinander beabstandeten Armen (28, 30) trennbar sind,
**dadurch gekennzeichnet, dass**
A) die Verriegelungskappe (40) eine zweistufige Verriegelungskappe (40) ist, welche ein aussen gewindetes äusseres Teilstück (42) mit einer inneren Bohrung (42a) zur gewindeten Aufnahme eines aussen gewindeten inneren Teilstücks (44) umfasst, wobei bei einer Rotation der Kappe (40) das äussere Teilstück (42) der Verriegelungskappe (40) die Büchse (36) verriegelt, während das innere Teilstück (44) der Verriegelungskappe (40) den Stab (45) verriegelt;
B) der Körper (20) zusätzlich eine Hülse (35) umfasst, wobei die Hülse (35) und die Büchse (36) verschiebbar in der Längsbohrung angeordnet sind und mindestens ein Abschnitt der Büchse (36) gleitbar in der Hülse (35) angeordnet ist; und
C) die Büchse (36) einen inneren sphärischen Hohlraum zur gleitbaren Aufnahme eines sphärischen Schraubenkopfs umfasst.

2. Knochenfixationselement nach Anspruch 1, **dadurch gekennzeichnet, dass** die Reduktionslappen einstückig mit den voneinander beabstandeten Armen ausgebildet sind.

3. Knochenfixationselement nach Anspruch 2, **dadurch gekennzeichnet, dass** die inneren und äusseren Sollbruchbereiche Nuten sind.

4. Knochenfixationselement nach Anspruch 1, **dadurch gekennzeichnet, dass** die inneren und äusseren Sollbruchbereiche (150) in der Form von Nuten nicht auf der gleichen Höhe sind und vorzugsweise der äussere Sollbruchbereich distal relativ zu dem inneren Sollbruchbereich angeordnet ist.

5. Knochenfixationselement nach Anspruch 1, **dadurch gekennzeichnet, dass** die inneren gewindeten Oberflächen ein negatives Gewindeprofil haben.

6. Knochenfixationselement nach Anspruch 1, zusätzlich umfassend:
einen Knochenanker, welcher polyaxial in einer Längsbohrung angeordnet ist, die sich von einer im Körper ausgebildeten oberen Öffnung zu einer im Körper ausgebildeten unteren Öffnung erstreckt.

7. Knochenfixationselement nach Anspruch 1, zusätzlich umfassend einen Knochenanker (12) mit einem erweiterten, sphärisch geformten Kopfabschnitt (14), welcher dem Knochenanker (12) ermöglicht, relativ zu der Büchse (36) polyaxial zu rotieren.

8. Knochenfixationselement nach Anspruch 7, **dadurch gekennzeichnet, dass** die Büchse einen inneren kugelschichtförmigen Hohlraum zur polyaxialen Aufnahme eines erweiterten Kopfabschnitts des Knochenankers umfasst.

9. Knochenfixationselement nach Anspruch 7, **dadurch gekennzeichnet, dass** die Längsbohrung benachbart zur unteren Öffnung eine kugelschichtartige Kontaktfläche zur Aufnahme einer äusseren kugelschichtartigen Fläche auf der Büchse umfasst, so dass die Büchse relativ zum Körper polyaxial rotierbar ist.

10. Knochenfixationselement nach Anspruch 9, **dadurch gekennzeichnet, dass** die Büchse ein unteres Ende umfasst, wobei sich das untere Ende der Büchse durch die untere Öffnung des Körpers ausdehnt, wenn die Verschlusskappe in Gewindeeingriff mit den inneren gewindeten Oberflächen des Körpers ist.

11. Knochenfixationselement nach Anspruch 1, zusätzlich umfassend:
eine Kappe, welche an einem proximalen Ende der Reduktionslappen montiert ist.

12. Knochenfixationselement nach Anspruch 11, **dadurch gekennzeichnet, dass** die Kappe eine Schulter umfasst.

13. Knochenfixationselement nach Anspruch 11, **dadurch gekennzeichnet, dass** die Kappe eine Antriebsoberfläche für einen Eingriff eines Antriebswerkzeugs zum Aufbringen einer Gegendrehkraft umfasst.

14. Knochenfixationselement nach Anspruch 11, **dadurch gekennzeichnet, dass** die inneren und äusseren Sollbruchbereiche ein Paar von Verbindungspunkten auf beiden Seiten eines durchgehenden Schlitzes umfassen wobei sich der durchgehende Schlitz, von der äusseren Oberfläche zur inneren Oberfläche erstreckt.

## Revendications

1. Elément de fixation osseuse (10) permettant de relier une tige (45) à l'os d'un patient, l'élément de fixation osseuse (10) comprenant :
un corps (20) comportant des bras (28, 30) espacés l'un de l'autre, définissant entre eux un canal (26) recevant une tige, chacun des bras (28, 30) comprenant une patte de réduction (100, 102) s'étendant à partir de chacun de ces bras, les pattes de réduction (100, 102) étant séparées des bras (28, 30) par des régions de rupture intérieure et extérieure (150), la région de rupture intérieure étant formée sur la surface intérieure du corps (20) et la région de rupture extérieure formée sur la surface extérieure du corps (20), des surfaces filetées intérieures (121) étant formées sur la longueur de la surface intérieure du corps (20), le corps (20) ayant un perçage longitudinal s'étendant à partir d'une ouverture du haut formée dans le corps (20), jusqu'à une ouverture du bas formée dans le corps (20);
une douille (36) insérée dans le perçage longitudinal, comprenant une extrémité inférieure, et
un capuchon de verrouillage (40) fileté extérieurement pouvant s'engager par filetage avec les surfaces filetées intérieures, les pattes de réduction (100, 102) étant séparables des bras (28, 30) espacés l'un de l'autre, au niveau des régions de rupture intérieure et extérieure (150),
**caractérisé en ce que**
A) le capuchon de verrouillage (40) est un capuchon de verrouillage (40) à deux étages comprenant une partie extérieure (42) filetée extérieurement comportant un perçage interne (42a) permettant de recevoir, par filetage, une partie intérieure (44) filetée extérieurement, faisant que, lors de la rotation du capuchon (40), la partie extérieure (42) du capuchon de verrouillage (40) verrouille la douille (36), tandis que la partie intérieure (44) du capuchon de verrouillage (40) verrouille la tige (45) ;
B) le corps (20) comprend en outre un manchon (35), le manchon (35) et la douille (36) étant disposés en coulissant à l'intérieur du perçage longitudinal, au moins une partie de la douille (36) étant disposée en coulissant à l'intérieur du manchon (35) ; et
C) la douille (36) comporte une cavité sphérique intérieure pour recevoir, de façon coulissante, une tête de vis sphérique.

2. Elément de fixation osseuse selon la revendication 1, dans lequel les pattes de réduction sont formées en étant solidaires des bras espacés l'un de l'autre.

3. Elément de fixation osseuse selon la revendication 2, dans lequel les régions de rupture intérieure et extérieure sont des rainures.

4. Elément de fixation osseuse selon la revendication 1, dans lequel les régions de rupture intérieure et extérieure (150), se présentant sous la forme de rainures, ne sont pas au même niveau, et, de préférence, la région de rupture extérieure est située de façon distale par rapport à la région de rupture intérieure.

5. Elément de fixation osseuse selon la revendication 1, dans lequel les surfaces filetées intérieures ont un profil de filetage négatif.

6. Elément de fixation osseuse selon la revendication 1, comprenant en outre :
une ancre osseuse disposée de façon polyaxiale à l'intérieur d'un perçage longitudinal s'étendant à partir d'une ouverture du haut formée dans le corps, jusqu'à une ouverture du bas formée dans le corps.

7. Elément de fixation osseuse selon la revendication 1, comprenant en outre une ancre osseuse (12) comportant une partie (14) élargie et de forme sphérique, formant la tête, permettant à l'ancre osseuse (12) de tourner de façon polyaxiale par rapport à la douille (36).

8. Elément de fixation osseuse selon la revendication 7, dans lequel la douille comprend une cavité tronco-sphérique intérieure pour recevoir, de façon polyaxiale, une partie élargie formant la tête de l'ancre osseuse.

9. Elément de fixation osseuse selon la revendication 7, dans lequel le perçage longitudinal comprend une surface de contact tronco-sphérique adjacente à l'ouverture du bas, pour recevoir une surface tronco-sphérique extérieure formée sur la douille, de sorte que la douille peut tourner de façon polyaxiale par rapport au corps.

10. Elément de fixation osseuse selon la revendication 9, dans lequel la douille comprend une extrémité inférieure, l'extrémité inférieure de la douille s'étendant à travers l'ouverture du bas du corps quand le capuchon de verrouillage est engagé par filetage avec les surfaces filetées intérieures du corps.

11. Elément de fixation osseuse selon la revendication 1, comprenant en outre
un capuchon monté au niveau d'une extrémité proximale des pattes de réduction.

12. Elément de fixation osseuse selon la revendication 11, dans lequel le capuchon comprend un épaulement.

13. Elément de fixation osseuse selon la revendication 11, dans lequel le capuchon comprend une surface de guidage pour engager un outil de guidage servant à appliquer une force de couple antagoniste.

14. Elément de fixation osseuse selon la revendication 11, dans lequel les régions de rupture intérieure et extérieure sont constituées d'une paire de points d'assemblage placés de chaque côté d'une fente traversante, la fente traversante s'étendant à partir de la surface extérieure jusqu'à la surface intérieure.
